(19) Europäisches Patentamt
European Patent Office
Office européen des brevets

(11) Veröffentlichungsnummer: **0 038 471**
B1

# (12) EUROPÄISCHE PATENTSCHRIFT

(45) Veröffentlichungstag der Patentschrift: **14.11.84**

(21) Anmeldenummer: **81102653.3**

(22) Anmeldetag: **08.04.81**

(51) Int. Cl.³: **C 07 C 49/203,** C 07 C 49/217, C 07 C 45/68, A 61 K 7/46, A 23 L 1/226, C 11 B 9/00

## (54) Neue beta-gamma-ungesättigte Ketone und isoprenoide 2,6-Dienone, Verfahren zu deren Herstellung und ihre Verwendung als Geruchs- und Geschmacksstoffe.

(30) Priorität: **09.04.80 DE 3013565**

(43) Veröffentlichungstag der Anmeldung: **28.10.81 Patentblatt 81/43**

(45) Bekanntmachung des Hinweises auf die Patenterteilung: **14.11.84 Patentblatt 84/46**

(84) Benannte Vertragsstaaten: **BE CH DE FR GB IT LI NL**

(56) Entgegenhaltungen:
**DE - A - 2 256 347**
**DE - C - 1 244 784**
**US - A - 4 169 109**

**Chemical Abstracts Band 63, Nr. 12, 1965, Columbus, Ohio, USA, J.M. CONIA et al. "Thermal cyclization of a,b,e,z-diethylenic ketones", Spalte 16229e**
**Chemical Abstracts Band 71, Nr. 5, 1969, Columbus, Ohio, USA, P. MARTINET et al. "Activated montmorillonite as a catalyst in the synthesis of cyclic acetals", Seite 322, Spalte 1, Abstract Nr. 22048q**

(73) Patentinhaber: **Consortium für elektrochemische Industrie GmbH, Zielstattstrasse 20, D-8000 München 70 (DE)**

(72) Erfinder: **Gebauer, Helmut, Dr. Dipl.-Chem., Schaffhauser Strasse 18/VII, D-8000 München 71 (DE)**
Erfinder: **Hafner, Walter, Dr. Dipl.-Chem, Sommerfeld 15, D-8024 Furth (DE)**

**EP 0 038 471 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue α-tertiäre,β-γ-olefinisch ungesättigte Ketone, isoprenoide 2,6-Dienone bzw. deren, durch Anlagerung von Wasserstoff an mindestens eine olefinische oder carbonylische Doppelbindung modifizierte Abkömmlinge, Verfahren zu deren Herstellung sowie ihre Verwendung als Geruchs- und Geschmacksstoffe.

In der DE-PS 12 44 784 wird ein Verfahren zur Herstellung von in α-Stellung alkylierten, auch als Geruchsstoffe verwendbaren, Ketonen vorgestellt, nach dem Ketone mit organischen Halogeniden in Gegenwart von Alkalihydroxyd und katalytischen Mengen an Stickstoffbase in α-Stellung alkyliert werden. Das reaktive Zentrum am Keton ist α-ständiger Wasserstoff. Derartige Herstellungsverfahren liefern jedoch im allgemeinen Substanzgemische, da Ketone zumeist mehrere α- bzw. α'-ständige Wasserstoffatome besitzen. Darüberhinaus wurde das hier vorgeschlagene Verfahren, nach dem einige der erfindungsgemässen Verbindungen im Prinzip zugänglich wären, derart ungewöhnlich substituierte Ausgangsverbindungen erfordern, dass dieser Syntheseweg für die erfindungsgemässen Verbindungen praktisch ausscheidet und sie deshalb auch nicht nahelegt.

Ferner berichten J.M. Conia et al. in Bull. Soc. chim. France, 1966 (1), Seiten 273 bis 277, von einer Allylierungsreaktion des α-substituierten, α-β-ungesättigten Ketons 3,4-Dimethyl-pent-3-en-2-on mit Allylbromid in Gegenwart von Natrium-tert.-amylat in homogener Phase zu 3-iso-Propenyl-3-Methyl-Hex-5-en-2-on und dessen weitere Umwandlung zu einem cis-trans-Isomerengemisch der 3,4-Dimethyl-octa-4,7-dien-2-one durch thermische Behandlung bei 230°C. Nachteiligerweise müssen für die Allylierungsreaktion äquimolare Mengen an Natrium-tert.-Amylat eingesetzt werden, deren Herstellung den problematischen Umgang mit metallischem Natrium erfordert.

Ferner veröffentlichten Kiese et al. in Yugaku 26, 474 (1977) Ref. CA 87, 133818 die phasentransferkatalysierte Reaktion von Aceton und Prenylchlorid zu 6-Methyl-hept-5-en-2-on. Alkylierungsreaktionen an Ketonen, die an α-ständigem Wasserstoff angreifen, liefern jedoch, wie bereits oben ausgeführt, nur in solchen Ausnahmefällen einheitliche Produkte, bei denen von Ketonen mit nur einem einzigen α-ständigen Wasserstoff ausgegangen wird.

Die DE-OS 2 256 342 lehrt ein Verfahren zur Herstellung von α-β, e-ε-ungesättigten Ketonen, die zumindest eine neun Glieder umfassende Kohlenstoffkette aufweisen. Diese Verbindungen sind durch Umsetzen von allylischem Alkohol mit Butadienylethern zugänglich. Sie dienen als Vor- und Zwischenprodukte für die Herstellung von Farb- und Riechstoffen. Demgegenüber besitzen die erfindungsgemässen Ketone den Vorteil, dass sie chemisch wesentlich leichter zugänglich sind und als solche als Geruchs- und Geschmacksstoffe einsetzbar sind.

In US-PS 4 169 109 wird ein Verfahren zur Herstellung von α-β-ungesättigten Ketonen oder α-β, γ-δ-ungesättigten Ketonen durch Kondensation von Aldehyden mit Ketonen in Gegenwart von Zinkacetat beschrieben. Es handelt sich im Prinzip um Aldolkondensationsprodukte, von denen eine Auswahl auch als Duftstoffe Verwendung finden. Erfindungsgemässe Verbindungen werden nicht erwähnt. Der vorgeschlagene Syntheseweg ist auch für die erfindungsgemässen Verbindungen ungeeignet.

Weiterhin ist die Umsetzung aktivierter organische Halogenide, wie Allyl- oder Benzyl-Halogenide mit α-alkylierten, α-β-ungesättigten Aldehyden beschrieben (vgl. hierzu US-PS 4 010 207).

Es wurde nun gefunden, dass sich derartige Reaktionen auch auf, gegenüber Aldehyden wesentlich reaktionsträgere α-alkylierte, α-β-ungesättigte Ketone übertragen lassen. Es musste befürchtet werden, dass unter den Bedingungen der phasentransferkatalysierten Reaktion analog der oben zitierten Entgegenhaltung CA 87, 133818 r zumindest unter erheblichem Ausbeuteverlust an Zielprodukt auch α-ständiger Wasserstoff alkyliert wird. Überraschenderweise bleibt eine derartige Reaktion jedoch aus.

Gegenstand der Erfindung sind Verbindungen der allgemeinen Formel I

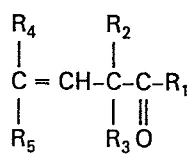

wobei

$R_1$ eine Alkyl- oder Alkoxyalkylgruppe mit 1 bis 6 Kohlenstoffatomen, oder eine, ggf. maximal 2 weitere Substituenten tragende Phenylgruppe darstellt, wobei die Substituenten bis zu 2 Alkyl- oder Alkoxyalkylgruppen mit 1 bis 3 Kohlenstoffatomen und/oder bis zu 2 Halogenatome sein können,

$R_2$ einen Alkyl- oder Alkoxyalkylrest mit 1 bis 5 Kohlenstoffatomen oder eine, ggf. maximal 2 weitere Substituenten tragende Phenylgruppe bedeutet, wobei die Substituenten bis zu 2 Alkyl- oder Alkoxyalkylgruppen mit 1 bis 3 Kohlenstoffatomen und/oder bis zu 2 Halogenatome sein können, oder

$R_1$ und $R_2$ zusammen einen Ring mit 4 bis 7 Kohlenstoffatomen bilden,

$R_3$ ein allylischer Rest mit 3 bis 5 Kohlenstoffatomen, oder ein, am aromatischen Ring ggf. maximal 2 weitere Substituenten tragender Benzylrest ist, wobei die Substituenten bis zu 2 Alkyl- oder Alkoxyalkylgruppen mit 1 bis 3 Kohlenstoffatomen und/oder bis zu 2 Halogenatome sein können, und

$R_4$ und $R_5$ Wasserstoff, oder Alkyl- bzw. Alkoxyalkylreste mit 1 bis 6 Kohlenstoffatomen, Alkenylreste mit 2 bis 6 Kohlenstoffatomen oder, ggf. bis zu 2 weitere Substituenten tragende Phenylgruppen darstellen, wobei die

Substituenten bis zu 2 Alkyl- oder Alkoxyalkylgruppen mit 1 bis 3 Kohlenstoffatomen und/oder bis zu 2 Halogenatome sein können.

Besonders bevorzugt sind solche Verbindungen der allgemeinen Formel I, für die $R_3$ den Allyl- oder Methallylrest bedeutet.

Die Verbindungen besitzen in $\alpha$-Stellung zur Carbonylfunktion einen tertiären Kohlenstoff, sind in $\beta$-$\gamma$-Stellung olefinisch ungesättigt und weisen ebenso in der $\gamma'$-$\delta'$-Stellung (gemäss $R_3$) eine olefinische Struktureinheit auf, die ggf. durch eine aromatische Struktureinheit ersetzt sein kann. Ferner besitzt das $\beta$-Kohlenstoffatom vinylischen Wasserstoff.

Weiterhin sind Gegenstand der Erfindung Verbindungen der allgemeinen Formel II

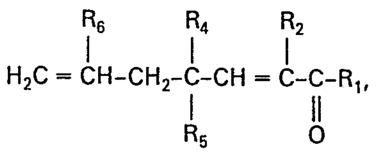

erhältlich durch Temperaturbehandlung von Verbindungen der allgemeinen Formel I, für die $R_3$ den Allyl- oder Methallylrest bedeutet, bei 80 bis 300°C,

wobei

- $R_1$ eine Alkyl- oder Alkoxyalkylgruppe mit 1 bis 6 Kohlenstoffatomen oder eine, ggf. maximal 2 weitere Substituenten tragende Phenylgruppe darstellt, wobei die Substituenten bis zu 2 Alkyl- oder Alkoxyalkylgruppen mit 1 bis 3 Kohlenstoffatomen und/oder bis zu 2 Halogenatome sein können,
- $R_2$ einen Alkyl- oder Alkoxyalkylrest mit 1 bis 5 Kohlenstoffatomen oder eine, ggf. 2 weitere Substituenten tragende Phenylgruppe bedeutet, wobei die Substituenten bis zu 2 Alkyl- oder Alkoxyalkylgruppen mit 1 bis 3 Kohlenstoffatomen und/oder bis zu 2 Halogenatome sein können, oder
- $R_1$ und $R_2$ zusammen einen Ring mit 4 bis 7 Kohlenstoffatomen bilden,
- $R_4$ und $R_5$ Wasserstoff, oder Alkyl- bzw. Alkoxyalkylreste mit 1 bis 6 Kohlenstoffatomen, Alkenylreste mit 2 bis 6 Kohlenstoffatomen oder, ggf. bis zu 2 weitere Substituenten tragende Phenylgruppen bedeuten, wobei die Substituenten bis zu 2 Alkyl- oder Alkoxyalkylgruppen mit 1 bis 3 Kohlenstoffatomen und/oder bis zu 2 Halogenatome sein können, und
- $R_6$ für Wasserstoff oder Methyl steht.

Einen weiteren Gegenstand der Erfindung bilden solche Verbindungen, die sich aus den allgemeinen Formeln I und II in der Weise herleiten, dass sie durch Anlagerung von Wasserstoff an mindestens eine olefinische oder carbonylische Doppelbindung modifiziert sind.

Ein bevorzugtes Verfahren zur Herstellung der Verbindungen der allgemeinen Formel I ist dadurch gekennzeichnet, dass Verbindungen der allgemeinen Formel

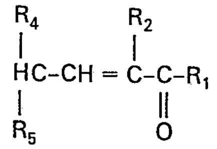

mit Verbindungen der allgemeinen Formel

$R_3$-Hal

wobei -Hal für Chlorid, Bromid oder Jodid steht, in einem organisch/alkalischen 2-Phasensystem in Gegenwart eines Phasentransferkatalysators umgesetzt werden.

Die Reaktionstemperaturen betragen dabei im allgemeinen zwischen 0 und 150°C, bevorzugt 20 bis 110°C. Oftmals wird ein optimales Verhältnis von Reaktionszeit und Ausbeute bei Temperaturen zwischen 60 und 70°C erzielt.

In einer vorteilhaften Ausführungsform des Verfahrens wird das 2-Phasensystem mit dem Katalysator vorgelegt und ein Gemisch der Reaktionskomponenten zugetropft.

Es werden äquimolare Mengen an Keton und $R_3$-Hal (im folgenden als Alkylierungsmittel bezeichnet) benötigt. Zur Erzielung optimaler Ausbeuten wird jedoch zumeist ein Überschuss an Alkylierungsmittel eingesetzt, da in einer Nebenreaktion geringe Mengen an Alkylierungsmittel in den entsprechenden Alkohol oder Äther überführt werden.

Die Zutropfzeiten variieren zumeist, je nach Substrat, Katalysatormenge und Wärmetönung der Reaktion zwischen 10 Minuten und 5 Stunden. Oftmals gelingt es allein durch Einstellen einer entsprechenden Zutropfgeschwindigkeit der Reaktanten die Reaktionstemperatur zu kontrollieren.

Nach beendeter Substratzugabe lässt man ggf. zur Vervollständigung der Reaktion bei gleicher oder erhöhter Temperatur noch 1 bis 5 Stunden nachreagieren.

Die Aufarbeitung des Reaktionsgemisches kann durch konventionelle Techniken erfolgen: gewöhnlich trennt man die Phasen und unterwirft, zur Isolierung des gewünschten Produkts, die organische Phase einer fraktionierten Destillation.

Das 2-Phasensystem wird gebildet aus einem organischen, mit Wasser nicht mischbaren, inerten Lösungsmittel und einer 5- bis 50%igen, bevorzugt 20- bis 50%igen, wässrigen Lösung oder in fester Form vorliegendem Alkalimetallhydroxyd.

Beispiele für inerte Lösungsmittel sind Benzol, Toluol, Xylole, Cyclohexan, Petroläther, Benzine u.a. Es können ebenso Gemische eingesetzt werden.

Beispiele für Alkalihydroxyde sind NaOH, KOH u.a.

Bezogen auf eingesetztes Alkylierungsmittel sind äquimolae Mengen an Lauge erforderlich – reaktionsbeschleunigend wirkt jedoch ein etwa zweifacher Überschuss an Lauge.

Erfindungsgemäss können Phasentransferkatalysatoren eingesetzt werden, die auch bereits bisher zu derartigen Reaktionen verwendet wurden. Beispiele sind Kronenäther, quartäre Ammonium- und Phosphoniumsalze, insbesondere Tetra-butyl-ammonium-bromid. Die Katalysatoren werden in Mengen von 0,5 bis 5 Mol%, bezogen auf Alkylierungsmittel, eingesetzt, wobei sich 2 bis 3 Mol% in der Regel als am wirtschaftlichsten erweisen.

Die als Ausgangssubstanzen einzusetzenden Ketone sind beispielsweise als gekreuzte Aldolkondensationsprodukte von in α-Stellung zur Carbonylfunktion eine Methylengruppe enthaltenden Ketonen mit entsprechenden Aldehyden zugänglich.

Dieser Aldolkondensationsschritt kann ggf. zusammen mit dem Alkylierungsschritt in dem beschriebenen wässrig/alkalischen 2-Phasensystem im Eintopfverfahren durchgeführt werden. Diese Verfahrensweise kann insbesondere in solchen Fällen mit Vorteil angewendet werden, in denen von relativ reaktionsträgen Aldehyden (bei denen nicht die Bildung des unilateralen Aldolkondensationsprodukts überwiegt) und überschüssigem Keton ausgegangen wird.

Beispiele für erfindungsgemäss zu alkylierende Ketone sind Aldolkondensationsprodukte aus Methyl-ethyl-keton, Diethyl-keton, Phenyl-ethyl-keton, Methyl-propyl-keton, Dipropyl-keton, Phenylpropyl-keton, Methyl-butyl-keton, Cyclohexanon, Cyclopentanon und Acetaldehyd, Propionaldehyd, Butyraldehyd, Isobutyraldehyd, Valeraldehyd, Isovaleraldehyd, 3-Pentenal, 4-Pentenal u.a.

Als Alkylierungsmittel sind beispielhaft zu nennen: Allylchlorid, Allylbromid, Methallylchlorid, Crotylchlorid, 1-Chlor-1-methyl-propen-2, Prenylchlorid, Benzylchlorid, Methoxybenzylchlorid, Chlorbenzylchlorid, Diphenylmethylbromid u.a.

Beispiele für erfindungsgemässe Verbindungen der allgemeinen Formel I sind:
3-Allyl-3-Methyl-Pent-4-en-2-on, 3-Allyl-3-Ethyl-Pent-4-en-2-on, 3-Allyl-3-Propyl-Pent-4-en-2-on, 3-Allyl-3-i-Propyl-Pent-4-en-2-on, 3-Allyl-3-Butyl-Pent-4-en-2-on, 3-Methallyl-3-Methyl-Pent-4-en-2-on, 3-Methallyl-3-Ethyl-Pent-4-en-2-on, 3-Methallyl-3-Propyl-Pent-4-en-2-on, 3-Methallyl-3-i-Propyl-Pent-4-en-2-on, 3-Methallyl-3-Butyl-Pent-4-en-2-on, 3-Crotyl-3-Methyl-Pent-4-en-2-on, 3-Crotyl-3-Ethyl-Pent-4-en-2-on, 3-Allyl-3-Propyl-Pent-4-en-2-on, 3-Crotyl-i-Propyl-Pent-4-en-2-on, 3-Crotyl-Butyl-Pent-4-en-2-on, 3-Benzyl-3-Methyl-Pent-4-en-2-on, 3-Benzyl-3-Ethyl-Pent-4-en-2-on, 3-Benzyl-3-Propyl-Pent-4-en-2-on, 3-Benzyl-3-i-Propyl-Pent-4-en-2-on, 3-Benzyl-3-Butyl-Pent-4-en-2-on, 3-p-Methoxy-Benzyl-3-Methyl-Pent-4-en-2-on, 3-p-Methoxy-Benzyl-3-Ethyl-Pent-4-en-2-on, 3-p-Methoxy-Benzyl-3-Propyl-Pent-4-en-2-on, 3-p-Chlor-Benzyl-3-Methyl-Pent-4-en-2-on, 3-p-Chlor-Benzyl-3-Ethyl-Pent-4-en-2-on, 3-m,p-Di-chlor-Benzyl-3-Methyl-Pent-4-en-2-on, 3-Allyl-3-Methyl-Hex-4-en-2-on, 3-Allyl-3-Ethyl-Hex-4-en-2-on, 3-Allyl-3-Propyl-Hex-4-en-2-on, 3-Methallyl-3-Methyl-Hex-4-en-2-on, 3-Methallyl-3-Ethyl-Hex-4-en-2-on, 3-Methallyl-3-Propyl-Hex-4-en-2-on, 3-Crotyl-3-Methyl-Hex-4-en-2-on, 3-Crotyl-3-Ethyl-Hex-4-en-2-on, 3-Crotyl-3-Propyl-Hex-4-en-2-on, 3-Benzyl-3-Methyl-Hex-4-en-2-on, 3-Benzyl-3-Ethyl-Hex-4-en-2-on, 3-Benzyl-3-Propyl-Hex-4-en-2-on, 3-p-Methyl-Benzyl-Hex-4-en-2-on, 3-p-Methyl-Benzyl-3-Ethyl-Hex-4-en-2-on, 3-p-Methyl-Benzyl-Propyl-Hex-4-en-2-on, 5-Methyl-3-Allyl-3-Methyl-Hex-4-en-2-on, 3-Allyl-3-Methyl-Hept-4-en-2-on, 3-Allyl-3-Methyl-Octa-4-en-2-on, 3-Allyl-3-Methyl-Nona-4-en-2-on, 3-Allyl-3-Methyl-deca-4-en-2-on, 4-Allyl-4-Methyl-Hex-5-en-3-on, 5-Allyl-5-Methyl-Hept-6-en-4-on, 5-Allyl-5-Ethyl-Hept-6-en-4-on, 5-Allyl-5-Propyl-Hept-6-en-4-on, 4-Methallyl-4-Methyl-Hex-5-en-3-on, 4-Crotyl-4-Methyl-Hex-5-en-3-on, 4-Benzyl-4-Methyl-Hex-5-en-3-on, 2-Allyl-2-Vinyl-Cyclo-pentanon, 2-Allyl-2-Vinyl-Cyclo-hexanon, 2-Allyl-2-(But-2-enyl)-Cyclohexan-1-on, 3-Methyl-3-Prenyl-Pent-4-en-2-on, 3-Allyl-3-Methyl-Octa-4,7-dien-2-on, 3-Methyl-3-Prenyl-Hepta-4,6-dien-2-on, 3-Methyl-3-Allyl-5-Methoxy-Pent-4-en-2-on, 3-Methoxymethyl-3-Prenyl-Pent-4-en-2-on.

Das bevorzugte Herstellungsverfahren für Verbindungen der allgemeinen Formel II ist dadurch gekennzeichnet, dass Verbindungen der allgemeinen Formel I

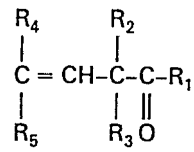

wobei $R_3$ für Allyl- oder Methallyl steht, einer Temperaturbehandlung von 80 bis 300°C, vorzugsweise 150 bis 220°C, unterworfen werden.

Die Reaktionen können unter Atmosphärendruck, also bei ca. 1 bar, in offenen Systemen ausgeführt werden. Gegebenenfalls ist es jedoch zweckmässig, im geschlossenen System bei Drücken von 0,5 bis 10 bar zu arbeiten. Massivere Reaktionsbedingungen bezüglich sowohl Druck als auch Temperatur sind, zur Vermeidung allzu langer Reaktionszeiten, insbesondere bei der Synthese solcher erfindungsgemässer Verbindungen angezeigt, die sperrigere Reste tragen. Ebenso ist es oftmals zweckmässig, die Umsetzungen unter Inertgasatmosphäre durchzuführen. Beispiele für Inertgase sind Stickstoff, Argon u.a.

Der Fortgang der Umlagerungen kann beispielsweise mit Hilfe sprektroskopischer oder chromatographischer Methoden, aber auch durch Erstellen einer Siedekurve verfolgt werden, da die umgelagerten Produkte in praktisch allen Fällen höhere Siedepunkte als die umzulagernden Substanzen aufweisen.

Erfindungsgemäss werden als Ausgangssubstanzen eine Auswahl der Verbindungen der allgemeinen Formel I eingesetzt, für die $R_3$ den Allyl- oder Methallylrest bedeutet. Diese Verbindungen gehen unter den oben beschriebenen Reaktionsbedingungen Umlagerungen zu Verbindungen der Formel II ein.

Beispiele für Verbindungen der allgemeinen Formel II sind:

3-Methyl-3,7-octadien-2-on, 3-Ethyl-3,7-octadien-2-on, 3-Propyl-3,7-octadien-2-on, 3-Butyl-3,7-octadien-2-on, 3-Pentyl-3,7-octadien-2-on, 3,7-Dimethyl-3,7-octadien-2-on, 3-Ethyl-7-Methyl-3,7-octadien-2-on, 3-Propyl-7-Methyl-3,7-octadien-2-on, 3-Butyl-7-Methyl-3,7-octadien-2-on, 3,5-Dimethyl-3,7-octadien-2-on, 3,5,5-Trimethyl-3,7-octadien-2-on, 3,5,5,7-Tetramethyl-3,7-octadien-2-on, 5-Ethyl-3,5,7-Trimethyl-3,7-octadien-2-on, 3-Methyl-5-5-Diethyl-3,7-octadien-2-on, 3,5,5-Triethyl-3,7-octadien-2-on, 3,5,5-Triethyl-7-Methyl-3,7-octadien-2-on, 3-Propyl-5,5-Diethyl-3,7-octadien-2-on, 3-Butyl-5,5-Dimethyl-3,7-octadien-2-on, 4-Methyl-4,8-nonadien-3-on, 4-Ethyl-4,8-nonadien-3-on, 4-Propyl-4,8-nonadien-3-on, 4,8-Dimethyl-4,8-nonadien-3-on, 4-Ethyl-8-Methyl-4,8-nonadien-3-on, 4-Propyl-8-Methyl-4,8-nonadien-3-on, 4,6-Dimethyl-4,8-nonadien-3-on, 4,6,8-Trimethyl-4,8-nonadien-3-on, 4,6,6,8-Tetramethyl-4,8-nonadien-3-on, 4,6-Diethyl-4,8-nonadien-3-on, 4,6,6-Triethyl-4,8-nonadien-3-on, 4,6,6-Triethyl-8-Methyl-4,8-nonadien-3-on, 4,6-Dimethyl-6-Ethyl-4,8-nonadien-3-on, 4,6-Dimethyl-6-Propyl-4,8-nonadien-3-on, 4,6,8-Trimethyl-6-Ethyl-4,8-nonadien-3-on, 2-(2-Methyl-Pent-4-enyliden)-Cyclohexan-1-on, 2-(2-Ethyl-Pent-4-enyliden)-Cyclohexan-1-on, 3-Methyl-5-Allyl-Octa-3,7-dien-2-on, 3-Methyl-5-Methoxy-Octa-3,7-dien-2-on, 3-Methoxymethyl-Octa-3,7-dien-2-on.

Weiterhin betrifft die Erfindung solche Verbindungen, die sich durch Anlagerung von Wasserstof an mindestens eine carbonylische oder olefinische Doppelbindung an Verbindungen der allgemeinen Formel I und II herleiten.

Die Anlagerung von Wasserstoff an mindestens eine Doppelbindung der erfindungsgemässen Verbindungen kann in an sich bekannter Weise erfolgen.

Beispiele für solche Hydrierungsagentien, die selektiv die Carbonylfunktion hydrieren, sind Natriumboranat, Lithiumalanat u.a.

Totalhydrierungen lassen sich beispielsweise mit molekularem Wasserstoff in Gegenwart von Raney-Nickel erzielen. Durch Einsatz weniger aktiver Katalysatoren der Platinmetalle lassen sich mit molekularem Wasserstoff ggf. selektive Hydrierungen ungeschützter Kohlenstoffdoppelbindungen durchführen, während solche Kohlenstoffdoppelbindungen, die durch Substituenten geschützt sind, erhalten bleiben.

Beispiele für, durch Anlagerung von Wasserstoff modifizierte Verbindungen der allgemeinen Formel I und II sind:

3-Allyl-3-Methyl-Pent-4-en-2-ol, 3-Allyl-3-Ethyl-Pent-4-en-2-ol, 3-Allyl-3-Propyl-Pent-4-en-2-ol, 3-Ethyl-3-Methyl-Hexan-2-ol, 3-Ethyl-3,3-Diethyl-Hexan-2-ol, 3-Methyl-3-Propyl-Hexan-2-ol, 3-Methallyl-3-Methyl-Pent-4-en-2-ol, 3-Ethyl-3,5-Dimethyl-Hexan-2-ol, 3,3-Diethyl-3-Methyl-Hexan-2-ol, 3-Ethyl-3-Methyl-3-Propyl-Hexan-2-ol, 3-Crotyl-3-Methyl-Pent-4-en-2-ol, 3-Crotyl-3-Ethyl-Pent-4-en-2-ol, 3-Benzyl-3-Methyl-Pent-4-en-2-ol, 3-Ethyl-3-Methyl-Heptan-2-ol, 3,3-Diethyl-Heptan-2-ol, 3-Benzyl-3-Methyl-Pent-4-en-2-ol, 3-Benzyl-3-Methyl-Pentan-2-ol, 3-Benzyl-3-Ethyl-Pentan-2-ol, 3-Allyl-3-Methyl-Hex-4-en-2-ol, 3-Allyl-3-Ethyl-Hex-4-en-2-ol, 3-Allyl-3-Propyl-Hex-4-en-2-ol, 3-Methyl-3-Propyl-Hexan-2-ol, 3-Ethyl-3-Propyl-Hexan-2-ol, 3,3-Dipropyl-3-Hexan-2-ol, 3-Methyl-3-Methallyl-Hex-4-en-2-ol, 3-Ethyl-3-Methallyl-Hex-4-en-2-ol, 3,5-Dimethyl-3-Propyl-Hexan-2-ol, 3-Ethyl-3-Propyl-5-Methyl-Hexan-2-ol, 3-Benzyl-3-Methyl-Hex-4-en-2-ol, 3-Benzyl-3-Methyl-Hexan-2-ol, 3-Allyl-3-Methyl-Hept-4-en-2-ol, 3-Allyl-3-Ethyl-Hept-4-en-2-ol, 3-Methyl-3-Propyl-Heptan-2-ol, 3-Ethyl-3-Propyl-Heptan-2-ol, 3,3-Dipropyl-3-Heptan-2-ol, 4-Methyl-4-Vinyl-Hept-6-en-3-ol, 4-ethyl-4-Methyl-Heptan-3-ol, 5-Methyl-5-Vinyl-oct-7-en-4-ol, 5-Ethyl-5-Methyl-octan-4-ol, 5-Ethyl-5-Vinyl-oct-7-en-4-ol, 5,5-Diethyl-octan-4-ol, 5-Propyl-5-Vinyl-oct-7-en-4-ol, 5-Ethyl-5-Propyl-octan-4-ol, 4,6-Dimethyl-4-Vinyl-Hept-6-en-3-ol, 4-Ethyl-4,6-Dimethyl-Heptan-3-ol, 4-Methyl-4-Vinyl-oct-3-ol, 4-Ethyl-4-Methyl-octan-3-ol, 2-Allyl-2-Vinyl-Cyclopentan-1-ol, 2-Ethyl-2-Propyl-Cyclopentan-1-ol, 2-Allyl-2-Vinyl-Cyclohexan-1-ol, 2-Ethyl-2-Propyl-Cyclohexan-1-ol, 3-Methyl-3-Prenyl-Pent-4-en-2-ol, 3-Allyl-3-Methyl-Octa-4,7-dien-2-ol, 3-Methyl-3-Prenyl-Hepta-4,6-dien-2-ol, 3-Methyl-3-Propyl-5-Methoxy-Pentan-2-ol, 3-Methoxymethyl-3-Prenyl-Pent-4-en-2-ol, 3-p-Methoxy-Benzyl-3-Methyl-Pentan-2-ol, 3-Methyl-3-Propyl-Octan-2-ol, 3-Methyl-3,7-octadien-2-ol, 3-Ethyl-3,7-octadien-2-ol, 3-Propyl-3,7-octadien-2-ol, 3,5-Dimethyl-3,7-octadien-2-ol, 3,7-Dimethyl-3,7-octadien-2-ol, 2,5,5-Trimethyl-3,7-octadien-2-ol, 3,5,5,7-Tetramethyl-3,7-octadien-2-ol, 3-Ethyl-7-Methyl-3,7-octadien-2-ol, 3-Propyl-7-Methyl-3,7-octadien-2-ol, 3-Methyl-5,5-Dimethyl-3,7-octadien-2-ol, 3,5,5-Trimethyl-octan-2-ol, 3,5,5,7-Tetramethyl-octan-2-ol, 3-Methyl-5,5-Diethyl-octan-2-ol, 4-Methyl-4,8-nonadien-3-ol, 4-Ethyl-4,8-nonadien-3-ol, 4,8-Dimethyl-4,8-nonadien-3-ol, 4-Propyl-8-Methyl-4,8-nonadien-3-ol, 4,6,8-Trimethyl-4,8-nonadien-3-ol, 4,6-Dimethyl-onan-3-ol, 4,6,6-Triethyl-nonan-3-ol, 4,6,8-Trimethyl-nonan-3-ol, 3-Methyl-5-Allyl-Octa-3,7-dien-2-ol, 3-Methyl-5-Propyl-Octan-2-ol, 3-Methyl-5-Methoxy-Octa-3,7-dien-2-ol, 3-Methoxymethyl-Octa-3,7-dien-2-ol, 2-(2-Ethyl-Pent-4-enyliden)-Cyclohexan-1-ol, 2-(2-Ethyl-Pentyl)-Cyclohexan-1-ol.

Die erfindungsgemässen Verbindungen finden Anwendung als Geruchs- und Geschmacksstoffe. Es sind beispielsweise frucht-, holz- oder kräuterartige Duft- und Geschmacksnoten erzielbar. Sie sind weiterhin als Zwischenprodukte für Pestizide und Pharmazeutika von Interesse. Schliesslich eignen sich solche erfindungsgemässen Verbindungen, die Mehrfachbindungen enthalten, als Monomere zur Herstellung wertvoller Polymerisate bzw. Kondensate.

Nach den erfindungsgemässen Verfahren wird es ermöglicht, neue, ungesättigte Ketone mit einer Kohlenstoffdoppelbindung in β-γ-Stellung

zur Carbonylfunktion selektiv herzustellen. Dabei können in der Regel grosstechnisch zugängliche Basischemikalien eingesetzt werden. Schliesslich können die allylisch substituierten Ketone in neue isoprenoide Systeme überführt werden.

Die Erfindung wird nun anhand von Beispielen näher erläutert:

Beispiel 1

3-Benzyl-3-Methyl-Pent-4-en-2-on

In einem 1 l-Vierhalskolben mit Rührer, Thermometer, Tropftrichter und Rückflusskühler werden 160 g 50%iger NaOH, 50 ml Wasser, 200 ml Toluol und 10 g Tetra-Butyl-Ammonium-Jodid vorgelegt und auf 60°C erwärmt. Unter kräftigem Rühren wird nun ein Gemisch aus 98 g (1 Mol) 3-Methyl-Pent-3-en-2-on und 158 g (1,25 Mol) Benzylchlorid so schnell zugetropft, dass eine Temperatur von 65 bis 70°C gehalten wird (Dauer ca. 1 Stunde). Danach wird noch eine Stunde am Rückfluss erhitzt.

Zur Aufarbeitung werden 100 ml Wasser zugegeben, die Phasen getrennt und die organische Phase nach Abziehen des Lösungsmittels über eine 30 cm-Vigreux-Kolonne fraktoniert destilliert.

Es werden 130 g (69% d.Th., bezogen auf eingesetztes Keton) an Zielprodukt erhalten.

Fraktion $Kp_{0,01}$ 85 bis 87°C, farbloses Öl. Geruch: süss, holzartig.

Beispiel 2

3-Methyl-3-Prenyl-Pent-4-en-2-on

In einem 500 ml-Vierhalskolben, analog Beispiel 1, werden 100 ml Toluol, 100 ml 50%iger NaOH und 10 g Tetra-Butyl-Ammonium-Jodid vorgelegt und auf 60°C erwärmt.

Ohne weiteres Erhitzen werden nun unter Rühren 52,3 g (0,5 Mol) Prenylchlorid und 49 g (0,5 Mol) 3-Methyl-Pent-3-en-2-on, im Gemisch innerhalb von 1,5 Stunden zudosiert. Die Reaktionsmischung wird noch 3 Stunden bei 60 bis 70°C gehalten.

Zur besseren Phasentrennung wird mit 100 ml Wasser verdünnt, die wässrige Phase einmal mit 50 ml Toluol extrahiert und die vereinigten organischen Phasen nach Abziehen des Lösungsmittels fraktioniert destilliert.

Die Ausbeute beträgt 46 g (55% d.Th.) an Zielprodukt.

Fraktion $Kp_{12}$ 84 bis 85°C, farblose Flüssigkeit. Geruch: lakritzartig.

Beispiel 3

3-Allyl-3-Methyl-Pent-4-en-2-on

Unter den in Beispiel 2 beschriebenen Bedingungen werden 49 g (0,5 Mol) 3-Methyl-Pent-3-en-2-on mit 51 ml (0,625 Mol) Allylchlorid umgesetzt.

Die Aufarbeitung durch Phasentrennung und fraktonierte Destillation erbringt 24 g (35% d.Th.) an Zielprodukt.

Fraktion $Kp_{12}$ 64 bis 65°C, farblose Flüssigkeit. Geruch: pfefferminz-terpen-artig.

Beispiel 4

3-Methyl-Octa-3,7-dien-2-on

100 g 3-Allyl-3-Methyl-Pent-4-en-2-on werden in einem 250 ml-Kolben mit Rückflusskühler unter Durchleiten von gasförmigem Stickstoff am Rückfluss gekocht. Innerhalb von 19 Stunden erhöht sich die Kolbentemperatur von 165 auf 180°C. Danach zeigt das GC-Spektrum eine vollständige Umlagerung an.

Die anschliessende Destillation erbringt 92,8 g (92,8% d.Th.) an Zielprodukt.

$Kp_{12}$ 79 bis 80°C, farblose Flüssigkeit.

Beispiel 5

3-Allyl-3-Methyl-Octa-4,7-dien-2-on

Es wird analog Beispiel 1 verfahren: Unter Rühren wird bei einer Temperatur von 60°C ein Gemisch aus 138 g (1 Mol) 3-Methyl-Octa-3,7-dien-2-on und 92 (1,2 Mol) Allylchlorid zudosiert. Die Reaktionsmischung wird noch 3 Stunden bei 70°C gehalten, anschliessend die Phasen getrennt, die organische Phase mit Natriumsulfat getrocknet und über eine 30 cm-Vigreux-Kolonne fraktioniert destilliert.

Die Ausbeute beträgt 110,5 g (62% d.Th., bezogen auf eingesetztes Keton).

Fraktion $Kp_{12}$ 103°C, farbloses Öl. Geruch: pfeffer-frucht-artig.

Beispiel 6

3-Methyl-5-Allyl-Octa-3,7-dien-2-on

In der im Beispiel 4 beschriebenen Apparatur werden 100 g 3-Allyl-3-Methyl-4,7-Octadien-2-on 30 Stunden auf 185 bis 190°C erhitzt. Anschliessend wird fraktioniert destilliert.

Die Ausbeute beträgt 83,4 g (83,4% d.Th.) an Zielprodukt.

Fraktion $Kp_{12}$ 107 bis 108°C, farbloses Öl. Geruch: fruchtartig.

Beispiel 7

3-Benzyl-3-Methyl-Pent-4-en-2-ol

In einem 250 ml-Zweihalskolben mit Tropftrichter und Rückflusskühler werden 1,1 g (0,029 Mol) $LiAlH_4$ in 100 ml absolutem Äther vorgelegt. Unter Rühren wird nun eine Lösung von 18,8 g (0,1 Mol) 3-Benzyl-3-Methyl-Pent-4-en-2-on (gemäss Beispiel 1), gelöst in 40 ml absolutem Äther, so schnell zugetropft, dass das Gemisch mässig siedet. Die Reaktionsmischung wird noch 1 Stunde auf Rückfluss gehalten, anschliessend mit Eiswasser hydrolysiert, mit 2 n-$H_2SO_4$ schwach angesäuert, die Phasen getrennt und die organische Phase nach Trocknen mit Natriumsulfat im Vakuum destilliert.

Die Ausbeute an Zielprodukt beträgt 14,9 g (78,4% d.Th.).

$Kp_{0,01}$ 88°C, farbloses Öl.

Beispiel 8

3-Allyl-3-Methyl-Pent-4-en-2-ol

Analog Beispiel 7 werden 27,6 g (0,2 Mol) 3-Allyl-3-Methyl-Pent-4-en-2-on (gemäss Beispiel 3) mit 2,2 g (0,058 Mol) $LiAlH_4$ reduziert.

Die Ausbeute beträgt 24 g, entsprechend 85,7% d.Th.

$Kp_{12}$ 67 bis 69°C, farbloses Öl.

Beispiel 9

3-Methyl-5-Allyl-Octa-3,7-dien-2-ol

Analog Beispiel 7 werden 17,8 g (0,1 Mol) 3-Methyl-5-Allyl-Octa-3,7-dien-2-on (gemäss Beispiel 6) mit 1,1 g (0,029 Mol) $LiAlH_4$ umgesetzt.

Die Ausbeute beträgt 15,2 g (das sind 84,4% d.Th.).

$Kp_{12}$ 115°C, farbloses Öl. Geruch: kiefernartig.

**Patentansprüche**

1. Verbindungen der allgemeinen Formel I

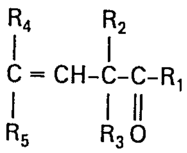

wobei

$R_1$ eine Alkyl- oder Alkoxyalkylgruppe mit 1 bis 6 Kohlenstoffatomen, oder eine, ggf. maximal 2 weitere Substituenten tragende Phenylgruppe darstellt, wobei die Substituenten bis zu 2 Alkyl- oder Alkoxyalkylgruppen mit 1 bis 3 Kohlenstoffatomen und/oder bis zu 2 Halogenatome sein können,

$R_2$ einen Alkyl- oder Alkoxyalkylrest mit 1 bis 5 Kohlenstoffatomen oder eine, ggf. maximal 2 weitere Substituenten tragende Phenylgruppe bedeutet, wobei die Substituenten bis zu 2 Alkyl- oder Alkoxyalkylgruppen mit 1 bis 3 Kohlenstoffatomen und/oder bis zu 2 Halogenatome sein können, oder

$R_1$ und $R_2$ zusammen einen Ring mit 4 bis 7 Kohlenstoffatomen bilden,

$R_3$ ein allylischer Rest mit 3 bis 5 Kohlenstoffatomen, oder ein am aromatischen Ring ggf. maximal 2 weitere Substituenten tragender Benzylrest ist, wobei die Substituenten bis zu 2 Alkyl- oder Alkoxyalkylgruppen mit 1 bis 3 Kohlenstoffatomen und/oder bis zu 2 Halogenatome sein können, und

$R_4$ und $R_5$ Wasserstoff, oder Alkyl- bzw. Alkoxyalkylreste mit 1 bis 6 Kohlenstoffatomen, Alkenylreste mit 2 bis 6 Kohlenstoffatomen oder, ggf. bis zu 2 weitere Substituenten tragende Phenylgruppen darstellen, wobei die Substituenten bis zu 2 Alkyl- oder Alkoxyalkylgruppen mit 1 bis 3 Kohlenstoffatomen und/oder bis zu 2 Halogenatome sein können.

2. Verbindungen nach Anspruch 1, wobei $R_3$ den Allyl- oder Methallylrest bedeutet.

3. Verbindungen der allgemeinen Formel II

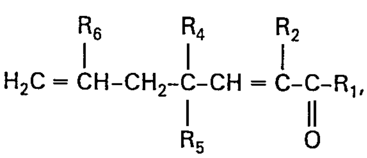

erhältlich durch Temperaturbehandlung von Verbindungen nach Anspruch 2 bei 80 bis 300°C, wobei

$R_1$ eine Alkyl- oder Alkoxyalkylgruppe mit 1 bis 6 Kohlenstoffatomen oder eine, ggf. 2 weitere Substituenten tragende Phenylgruppe darstellt, wobei die Substituenten bis zu 2 Alkyl- oder Alkoxyalkylgruppen mit 1 bis 3 Kohlenstoffatomen und/oder bis zu 2 Halogenatome sein können,

$R_2$ einen Alkyl- oder Alkoxyalkylrest mit 1 bis 5 Kohlenstoffatomen, oder eine, ggf. 2 weitere Substituenten tragende Phenylgruppe bedeutet, wobei die Substituenten bis zu 2 Alkyl- oder Alkoxyalkylgruppen mit 1 bis 3 Kohlenstoffatomen und/oder bis zu 2 Halogenatome sein können, oder

$R_1$ und $R_2$ zusammen einen Ring mit 4 bis 7 Kohlenstoffatomen bilden,

$R_4$ und $R_5$ Wasserstoff, oder Alkyl- bzw. Alkoxyalkylreste mit 1 bis 6 Kohlenstoffatomen, Alkenylreste mit 2 bis 6 Kohlenstoffatomen, oder, ggf. bis zu 2 weitere Substituenten tragende Phenylgruppen bedeuten, wobei die Substituenten bis zu 2 Alkyl- oder Alkoxyalkylgruppen mit 1 bis 3 Kohlenstoffatomen und/oder bis zu 2 Halogenatome sein können, und

$R_6$ für Wasserstoff oder Methyl steht.

4. Verbindungen nach einem der Ansprüche 1, 2 und 3, die durch Anlagerung von Wasserstoff an mindestens eine olefinische oder carbonylische Doppelbindung modifiziert sind.

5. Verfahren zur Herstellung von Verbindungen nach einem der Ansprüche 1 und 2, dadurch gekennzeichnet, dass Verbindungen der allgemeinen Formel

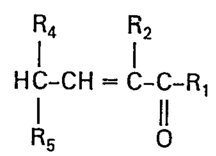

mit Verbindungen der allgemeinen Formel

$R_3$–Hal

wobei –Hal für Chlorid, Bromid oder Jodid steht, in einem organisch/alkalischen 2-Phasensystem in Gegenwart eines Phasentransfer-Katalysators umgesetzt werden.

6. Verfahren zur Herstellung von Verbindungen nach einem der Ansprüche 1 und 2, dadurch gekennzeichnet, dass Ketone der allgemeinen Formel

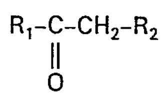

mit Aldehyden der allgemeinen Formel

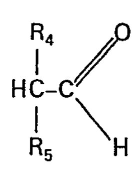

und mit Verbindungen der allgemeinen Formel

$R_3$-Hal

wobei -Hal für Chlorid, Brom oder Jodid steht, im Eintopfverfahren in einem organisch/alkalischen 2-Phasensystem in Gegenwart eines Phasentransfer-Katalysators umgesetzt werden.

7. Verfahren zur Herstellung von Verbindungen nach Anspruch 3, dadurch gekennzeichnet, dass Verbindungen nach Anspruch 2 einer Temperaturbehandlung von 80 bis 300°C unterworfen werden.

8. Verwendung von Verbindungen der Ansprüche 1, 2, 3 und 4 als Geruchs- und Geschmacksstoffe.

**Claims**

1. Compounds of the general formula I

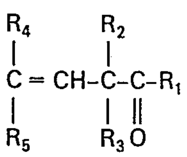

in which

$R_1$ represents an alkyl or alkoxyalkyl group having from 1 to 6 carbon atoms, or a phenyl group optionally carrying up to 2 further substituents, the said substituents being up to 2 alkyl or alkoxyalkyl groups having from 1 to 3 carbon atoms and/or up to 2 halogen atoms,

$R_2$ represents an alkyl or alkoxyalkyl radical having from 1 to 5 carbon atoms, or a phenyl group optionally carrying up to 2 further substituents, the said substituents being up to 2 alkyl or alkoxyalkyl groups having from 1 to 3 carbon atoms and/or up to 2 halogen atoms, or

$R_1$ and $R_2$ together form a ring having from 4 to 7 carbon atoms,

$R_3$ represents an allylic radical having from 3 to 5 carbon atoms, or a benzyl radical optionally carrying up to 2 further substituents in the aromatic ring, the said substituents being up to 2 alkyl or alkoxyalkyl groups having from 1 to 3 carbon atoms and/or up to 2 halogen atoms, and

$R_4$ and $R_5$ each represent hydrogen, an alkyl or alkoxyalkyl radical having from 1 to 6 carbon atoms, an alkenyl radical having from 2 to 6 carbon atoms, or a phenyl group optionally carrying up to 2 further substituents, the said substituents being up to 2 alkyl or alkoxyalkyl groups having from 1 to 3 carbon atoms and/or up to 2 halogen atoms.

2. Compounds according to claim 1, in which $R_3$ represents an allyl or methallyl radical.

3. Compounds of the general formula II

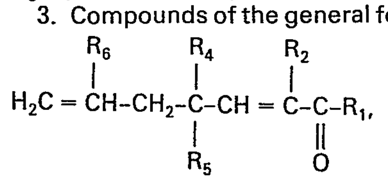

obtainable by heat treatment of compounds according to claim 2 at from 80 to 300°C, in which

$R_1$ represents an alkyl or alkoxyalkyl group having from 1 to 6 carbon atoms, or a phenyl group optionally carrying up to 2 further substituents, the said substituents being up to 2 alkyl or alkoxyalkyl groups having from 1 to 3 carbon atoms and/or up to 2 halogen atoms,

$R_2$ represents an alkyl or alkoxyalkyl radical having from 1 to 5 carbon atoms, or a phenyl group optionally carrying up to 2 further substituents, the said substituents being up to 2 alkyl or alkoxyalkyl groups having from 1 to 3 carbon atoms and/or up to 2 halogen atoms, or

$R_1$ and $R_2$ together form a ring having from 4 to 7 carbon atoms,

$R_4$ and $R_5$ each represent hydrogen, an alkyl or alkoxyalkyl radical having from 1 to 6 carbon atoms, an alkenyl radical having from 2 to 6 carbon atoms, or a phenyl group optionally carrying up to 2 further substituents, the said substituents being up to 2 alkyl or alkoxyalkyl groups having from 1 to 3 carbon atoms and/or up to 2 halogen atoms, and

$R_6$ represents hydrogen or methyl.

4. Compounds according to any one of claims 1, 2 and 3 that are modified by the addition of hydrogen to at least one olefinic or carbonylic double bond.

5. Process for the manufacture of compounds according to claim 1 or 2, characterised in that compounds of the general formula

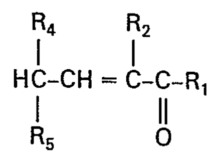

are reacted with compounds of the general formula

$R_3$-Hal

in which -Hal represents chloride, bromide or iodide, in an organic/alkaline two-phase system in the presence of a phase-transfer catalyst.

6. Process for the manufacture of compounds according to claim 1 or 2, characterised in that ketones of the general formula

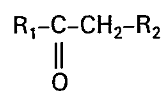

are reacted with aldehydes of the general formula

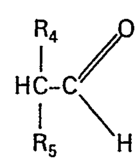

and with compounds of the general formula

$R_3$-Hal

in which -Hal represents chloride, bromide or iodide, in a one-pot process in an organic/alkaline two-phase system in the presence of a phase-transfer catalyst.

7. Process for the manufacture of compounds according to claim 3, characterised in that compounds according to claim 2 are subjected to heat treatment at from 80 to 300°C.

8. Use of compounds of claims 1, 2, 3 and 4 as perfumes and flavourings.

**Revendications**

1. Composés répondant à la formule générale (I):

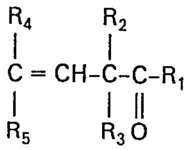

dans laquelle:

$R_1$ représente un radical alkyle ou alcoxyalkyle contenant de 1 à 6 atomes de carbone, ou un radical phényle portant éventuellement au plus deux substituants supplémentaires, lesquels peuvent être pris dans l'ensemble constitué par les radicaux alkyles et alcoxyalkyles contenant de 1 à 3 atomes de carbone et les atomes d'halogènes,

$R_2$ représente un radical alkyle ou alcoxyalkyle contenant de 1 à 5 atomes de carbone, ou un radical phényle portant éventuellement au plus deux substituants supplémentaires, lesquels peuvent être pris dans l'ensemble constitué par les radicaux alkyles et alcoxyalkyles contenant de 1 à 3 atomes de carbone et les atomes d'halogènes, ou encore

$R_1$ et $R_2$ forment ensemble un noyau contenant de 4 à 7 atomes de carbone,

$R_3$ représente un radical allylique contenant de 3 à 5 atomes de carbone, ou un radical benzyle dont le noyau aromatique porte éventuellement au plus deux substituants supplémentaires, lesquels peuvent être pris dans l'ensemble constitué par les radicaux alkyles et alcoxyalkyles contenant de 1 à 3 atomes de carbone et les atomes d'halogènes, et

$R_4$ et $R_5$ représentent chacun l'hydrogène, un radical alkyle ou alcoxyalkyle contenant de 1 à 6 atomes de carbone, un radical alcényle contenant de 2 à 6 atomes de carbone, ou un radical phényle portant éventuellement au plus deux substituants supplémentaires, lesquels peuvent être pris dans l'ensemble constitué par les radicaux alkyles et alcoxyalkyles contenant de 1 à 3 atomes de carbone et les atomes d'halogènes.

2. Composés selon la revendication 1 dans lesquels $R_3$ représente un radical allyle ou méthallyle.

3. Composés répondant à la formule générale (II):

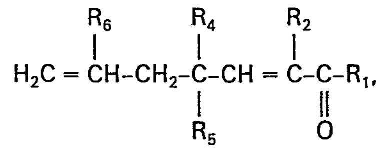

dans laquelle:

$R_1$ représente un radical alkyle ou alcoxyalkyle contenant de 1 à 6 atomes de carbone, ou un radical phényle portant éventuellement au plus deux substituants supplémentaires lesquels peuvent être pris dans l'ensemble constitué par les radicaux alkyles et alcoxyalkyles contenant de 1 à 3 atomes de carbone et les atomes d'halogènes,

$R_2$ représente un radical alkyle ou alcoxyalkyle contenant de 1 à 5 atomes de carbone, ou un radical phényle portant éventuellement au plus deux substituants supplémentaires, lesquels peuvent être pris dans l'ensemble constitué par les radicaux alkyles et alcoxyalkyles contenant de 1 à 3 atomes de carbone et les atomes d'halogènes, ou encore

$R_1$ et $R_2$ forment ensemble un noyau contenant de 4 à 7 atomes de carbone,

$R_4$ et $R_5$ représentent chacun l'hydrogène, un radical alkyle ou alcoxyalkyle contenant de 1 à 6 atomes de carbone, un radical alcényle contenant de 2 à 6 atomes de carbone, ou un radical phényle portant éventuellement au plus deux substituants supplémentaires, lesquels peuvent être pris dans l'ensemble constitué par les radicaux alkyles et alcoxyalkyles contenant de 1 à 3 atomes de carbone et les atomes d'halogènes, et

$R_6$ représente l'hydrogène ou un méthyle, que l'on peut obtenir en traitant à chaud, à une température de 80 à 300°C, des composés selon la revendication 2.

4. Composés selon l'une quelconque des revendications 1, 2 et 3, qui ont été modifiés par fixation d'hydrogène sur au moins une double liaison éthylénique ou carbonylique.

5. Procédé de préparation de composés selon l'une des revendications 1 et 2, procédé caractérisé en ce qu'on fait réagir des composés de formule générale:

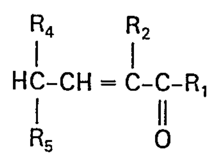

avec des composés répondant à la formule générale:

$R_3$-Hal

dans laquelle Hal représente le chlore, le brome ou l'iode, dans un système biphasé organique/

alcalin, en présence d'un catalyseur de transfert de phase.

6. Procédé de préparation de composés selon l'une des revendications 1 et 2, procédé caractérisé en ce qu'on fait réagir des cétones de formule générale:

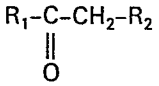

avec des aldéhydes de formule générale:

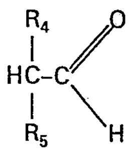

et avec des composés répondant à la formule générale:

$R_3$–Hal

dans laquelle Hal représente le chlore, le brome ou l'iode, par une méthode à un seul récipient, dans un système biphasé organique/alcalin, en présence d'un catalyseur de transfert de phase.

7. Procédé de préparation de composés selon la revendication 3, procédé caractérisé en ce qu'on soumet des composés selon la revendication 2 à un traitement par la chaleur à une température de 80 à 300°C.

8. Application de composés selon l'une quelconque des revendifications 1, 2, 3 et 4 comme parfums et aromatisants.